(19) Europäisches Patentamt
European Patent Office
Office européen des brevets

(11) Veröffentlichungsnummer: **0 058 326**
**B1**

(12) # EUROPÄISCHE PATENTSCHRIFT

(45) Veröffentlichungstag der Patentschrift: **06.06.84**

(51) Int. Cl.³: **C 07 C 45/61**, C 07 C 85/08, C 07 C 47/228, C 07 D 211/14, C 07 D 223/04, C 07 D 265/30

(21) Anmeldenummer: **82100670.7**

(22) Anmeldetag: **30.01.82**

(54) **Verfahren zur Herstellung von araliphatischen Aldehyden und/oder Aminen.**

(30) Priorität: **14.02.81 DE 3105446**

(43) Veröffentlichungstag der Anmeldung: **25.08.82 Patentblatt 82/34**

(45) Bekanntmachung des Hinweises auf die Patenterteilung: **06.06.84 Patentblatt 84/23**

(84) Benannte Vertragsstaaten: **CH DE FR GB LI NL**

(56) Entgegenhaltungen:
**EP - A - 0 007 093**
**EP - A - 0 017 893**
**CH - A - 474 464**

**Chemical Abstracts Band 91, Nr. 7, 13. August 1979 Columbus, Ohio, USA S.A. VOITKEVICH et al. "Synthesis *of beta-phenylpropionaldehyde and its aldol* condensation with aliphatic aldehydes" Seite 664, Spalte 1, Abstract Nr. 56543a**

(73) Patentinhaber: **BASF Aktiengesellschaft, Carl-Bosch-Strasse 38, D-6700 Ludwigshafen (DE)**

(72) Erfinder: **Gramlich, Walter, Dr., Auf der Hoehe 11, D-6803 Edingen (DE)**
Erfinder: **Heilen, Gerd, Dr., Schifferstadter Strasse 1 b, D-6720 Speyer (DE)**
Erfinder: **Mercker, Hans Jochen, Dr., Schwabenstrasse 14, D-6800 Mannheim (DE)**
Erfinder: **Siegel, Hardo, Dr., Hans-Purrmann-Allee 25, D-6720 Speyer (DE)**

**EP 0 058 326 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Es ist bekannt, daß man araliphatische Carbonylverbindungen in Gegenwart basischer Kondensationsmittel durch Umsetzung von aromatischen Aldehyden mit aliphatischen Carbonylverbindungen (Aldolkondensation) und anschließender Hydrierung herstellt [Houben-Weyl »Methoden der organischen Chemie« Band 7/1, S. 388 ff. und S. 76 ff (1954) sowie US-PS 1 844 013 (1929)]. Bei Verwendung aliphatischer Aldehyde erhält man araliphatische Aldehyde.

Es ist weiter bekannt, tertiäre Amine durch aminierende Hydrierung (Umsetzung von Carbonylverbindungen mit sekundären Aminen in Gegenwart von Wasserstoff und einem Hydrierkatalysator) herzustellen [Houben-Weyl »Methoden der organischen Chemie« Band 4/2, S. 328 ff, Band 11/1, S. 602 ff., S. 643 ff.].

Normalerweise geht man so vor, daß man zunächst die Aldolkondensation vornimmt, das Aldol isoliert und danach in einem weiteren Reaktionsschritt hydriert bzw. aminierend hydriert (vgl. US-PS 1 844 013).

Bei der technischen Durchführung dieses Verfahrens treten jedoch eine ganze Reihe von Schwierigkeiten auf.

Zunächst hat man es mit einem mehrstufigen Verfahren zu tun, d. h. einem Verfahren, dessen Einzelschritte in getrennten Reaktionsgefäßen vorgenommen werden. Vor allem wegen der Aufarbeitung (Destillation) der als Zwischenprodukte anfallenden $\alpha,\beta$-ungesättigten Aldehyde ist ein solches Verfahren weniger wirtschaftlich. Auch handelt es sich bei den in Rede stehenden $\alpha,\beta$-ungesättigten Aldehyden meist um hochschmelzende Verbindungen, die sowohl bei der Isolierung (Kristallisation beim Destillieren) als auch bei der Weiterverarbeitung unbequem zu handhaben sind, zumal sie in den gängigen organischen Lösungsmitteln nur schlecht löslich sind.

Ein weiterer, in jüngster Zeit immer wichtigerer Punkt ist die Toxikologie: Hier zählen $\alpha,\beta$-ungesättigte Carbonylverbindungen zu den toxischen Substanzen, während die entsprechend hydrierten Verbindungen als harmlos gelten.

Es wurde nun gefunden, daß sich die genannten Schwierigkeiten vermeiden lassen, wenn man bei absatzweisem Betrieb die Hydrierung bzw. aminierende Hydrierung in Gegenwart eines Palladiumkatalysators und alle Reaktionsschritte in einem einzigen Reaktionsgefäß ablaufen läßt, ohne ein Zwischenprodukt zu isolieren. Zu diesem Fall setzt man den Hydrierkatalysator von Anfang an, d. h. vor oder während der Aldolkondensation zu.

Dabei ist zunächst überraschend, daß die Aldolkondensation auch in Gegenwart des Hydrierkatalysators mit hohem Umsatz und hoher Selektivität abläuft. Ebenso überraschend ist, daß der Hydrierkatalysator die stark basische Reaktion des ursprünglichen Reaktionsgemisches aushält und sich nicht z. B. auflöst.

Zweckmäßig verfährt man so, daß der aliphatische Aldehyd in absatzweisem Betrieb unter Inertgas bei definierter Temperatur zum Gemisch aus Hydrierkatalysator, Lösungsmittel, Base und dem aromatischen Aldehyd zugefügt wird; das in situ entstandene Aldol wird dann nach mindestens teilweiser Neutralisation der Base direkt mit Wasserstoff unter Absättigung der CC-Doppelbindung weiter umgesetzt. Man verwendet in diesem Fall somit einen druckfesten Rührkessel o. ä.; nach Herauspumpen des schließlich flüssigen Reaktionsgemisches kann der zurückbleibende Katalysator erneut benutzt werden.

Bei fortlaufendem Betrieb ordnet man den Katalysator fest an, läßt das Aldehydgemisch vorreagieren und führt die Umsetzungsprodukte ohne weiteres fortlaufend (kontinuierlich) der Katalysatorschüttung zu.

Selbstverständlich läßt sich ein mit Flüssigkeitskreislauf betriebener Reaktor mit fest angeordnetem Katalysator auch absatzweise betreiben und verhält sich dann im Prinzip wie ein Rührkessel.

So entsteht bei der Umsetzung von 4-t-Butyl-benzaldehyd mit Propionaldehyd in Gegenwart basischer Katalysatoren 3[4-t-Butylphenyl]-2-methyl-2-propenal-1, das in fester Form (Schmelzpunkt 67° C) anfällt und geringe Löslichkeit zeigt; durch Hydrierung der Doppelbindung unter Erhalt der Aldehydfunktion läßt sich daraus der Riechstoff 3[4-t-Butylphenyl]2-methyl-1-propanal erhalten, der flüssig ist und keine Schwierigkeiten bei der Aufarbeitung (Herauspumpen, Destillation) sowie Handhabung bereitet.

Von den weiteren Möglichkeiten seien hier nur die beiden folgenden Kondensationsprodukte von p-substituierten Benzaldehyden mit Propionaldehyd erwähnt, die beide über 100° C schmelzen und aus Ethanol umkristallisiert werden können:

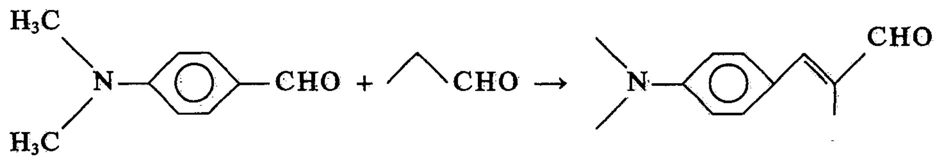

Fp. 108–115°C

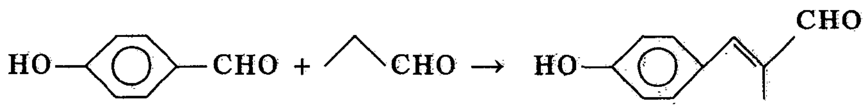

Fp. 170–182°C

Als aromatische Aldehyde können Benzaldehyd sowie einfach, zweifach und dreifach substituierte Benzaldehyde wie 2-Methyl-, 2,4-Dimethyl-, 3-Ethyl-, 4-Ethyl-, 2,4,6-Trimethylbenzaldehyd eingesetzt werden; besonders wichtige Aldehyde sind 4-t-Butylbenzaldehyd, 4-Isopropyl-benzaldehyd, Anisaldehyd, 2,4-Dichlorbenzaldehyd, 4-Fluorobenzaldehyd und 3-Phenoxybenzaldehyd.

Als aliphatische Aldehyde kommen z. B. Acetaldehyd, Propionaldehyd, Butyraldehyd oder 2-Ethylhexan-1-al in Betracht.

Als Lösungsmittel für das erfindungsgemäße Verfahren hat sich Methanol am besten bewährt, jedoch lassen sich genauso gut auch höhere Alkohole verwenden.

Als Basen haben sich bei dem erfindungsgemäßen Verfahren die Alkalihydroxide NaOH und KOH sowie Natriummethylat am besten bewährt.

Zur notwendigen Abstumpfung des pH-Wertes — andernfalls tritt bei Hydrierung Cannizzaro-Reaktion ein — sind organische sowie halogenfreie anorganische Säuren verwendbar. Bevorzugt verwendet man Carbonsäuren, so daß bei teilweiser Neutralisation eine gepufferte Lösung entsteht.

Als Hydrierkatalysator wird Palladium verwendet, das i. a. auf einem inerten Träger wie Aluminiumoxid, Titandioxid, Kohle usw. aufgetragen ist. Auch natürliche oder künstliche Silicate wie Aluminium-Silicate oder Magnesium-Silicat sind als Träger geeignet.

Auf die Träger können die Katalysatoren des erfindungsgemäßen Verfahrens aufgebracht werden, indem man sie z. B. mit Lösungen von Verbindungen der Katalysatoren behandelt; anschließend schlägt man dann die Metalle durch Reduktion der getränkten oder besprühten Salze nieder. Katalysatorzusätze lassen sich ebenfalls durch Tränkung oder durch Besprühung aufbringen. Ein günstiger Zusatz ist Zink, Cadmium oder Seltenerdmetalle bzw. deren Mischungen.

Die beste Reaktionstemperatur liegt im ersten Reaktionsschritt bei bis zu 50° C, bevorzugt zwischen 25 und 40° C; der Hydrierungsschritt verlangt i. a. Temperaturen von Raumtemperatur bis 120° C, bis höchstens 220° C.

Der benötigte Wasserstoffdruck hängt von der jeweiligen Verbindung ab und beträgt i. a. zwischen 5 und 200 bar, gelegentlich auch weniger.

Die Hydrierung kann in Gegenwart eines sekundären Amins vorgenommen werden, d. h., daß nach der Kondensation ein sekundäres Amin zugegeben wird und dann Hydrierung der CC-Doppelbindung, Enaminbildung und weitere Hydrierung nebeneinander ablaufen.

Als sekundäre Amine kommen sowohl N-dialkylierte Amine wie Dimethylamin, Diethylamin, Methylbutylamin, cyclische Amine wie Piperidin als auch cyclische Amine mit Heteroatomen wie Morpholin in Frage, die zusätzlich noch Substituenten tragen können wie z. B. 2,6-Dimethylmorpholin.

Zur Aufarbeitung wird das Reaktionsgemisch am einfachsten aus den Autoklaven z. B. über ein Steigrohr mit Fritte herausgepreßt und der zurückbleibende Hydrierkatalysator erneut eingesetzt.

Die Durchführbarkeit der Erfindung wurde u. a. mit den nachfolgenden Experimenten untersucht:

## Beispiel 1

In einem Autoklaven werden 30 l Methanol, 20 kg (123,5 Mol) 4-t-Butylbenzaldehyd und 2 kg eines Katalysators, der 0,5 Gew.-% Palladium, 5 Gew.-% Praseodym (berechnet als Oxid) auf $\gamma$-$Al_2O_3$ enthält, unter Stickstoff vorgelegt, 1,2 kg 50%ige wäßrige Natronlauge während 10 min zugegeben und bei 25 bis 30° C während 6 Stunden 7,88 kg (135,8 Mol) Propionaldehyd zugesetzt. Man rührt 30 Minuten bei 40° C nach, versetzt mit 700 g Eisessig, preßt Wasserstoff bis zu einem Druck von 2 bar auf und erhitzt auf 110° C. Danach wird bei einem Wasserstoffdruck von 17 bar und 50 Nl/h Abgas hydriert. Nach 6 bis 8 Stunden ist die Reaktion beendet.

Man entfernt die Lösung und läßt den Katalysator im Autoklav zurück. Nach fünfmaliger Wiederholung mit der selben Katalysatorfüllung war kein Aktivitätsverlust festzustellen. Durch Destillation bei 6 mbar und 126°C erhält man 22,1 g 3[4-t-Butylphenyl]-2-methylpropanal [88%, bezogen auf den Benzaldehyd].

## Beispiel 2

366 g Methanol, 55 g Katalysator (0,5 Gew.% Palladium auf Rutil) und 366 g (2,25 Mol) 4-t-Butylbenzaldehyd werden in einem 2 l-Autoklaven unter Stickstoff vorgelegt. Nach Zugabe von 12,5 g (0,31 Mol) Natronlauge werden innerhalb von 6 Stunden 140 g (2,4 Mol) Propionaldehyd zugesetzt, wobei die Temperatur während den ersten 4 Stunden zwischen 25 und 30° C, während den beiden letzten dann bei 40° C gehalten wird. Bei der gleichen Temperatur wird 10 min nachgerührt, mit 11,2 g (0,18 Mol) Eisessig versetzt und während 20 min 229 g (2,03 Mol) 2,6-Dimethylmorpholin zugesetzt. Jetzt wird 4 Stunden bei 40° C und 50 bar Wasserstoffdruck, 4 Stunden bei 100° C und 50 bar Wasserstoffdruck sowie 2 Stunden bei 100° C und 90 bar Wasserstoffdruck hydriert.

Nach dem Abkühlen wird das Reaktionsgemisch über eine Fritte abgezogen, so daß der Katalysator im Reaktionsraum zurückbleibt, das Lösungsmittel abdestilliert und der Rückstand unter vermindertem Druck destilliert. Als erste Fraktion ($KP_{0,01}$ bis 115° C) geht ein Gemisch aus 4-t-Butylbenzalkohol und N[4-t-Butylbenzyl]2,6-dimethylmorpholin über; die zweite Fraktion liefert dann 556 g 98%iges Zielprodukt (entspricht 1,8 Mol; 80% Selektivität bez. auf 4-t-Butylbenzaldehyd, 88% Selektivität, bez. auf 2,6-Dimethylmorpholin). Der im Autoklaven zurückgebliebene Katalysator wird weiter benutzt.

## Beispiel 3

Man legt in einem 1,2 l-Autoklaven 150 g Methanol, 20 g Katalysator (0,5% Pd auf Anatas) und 203 g (1,25 Mol) 4-t-Butylbenzaldehyd vor, versetzt mit 9,8 g (0,17 Mol) Kalilauge und setzt während 4 Stunden 72,5 g (1,25 Mol) Propionaldehyd unter schnellem Rühren bei 25 bis 30° C zu. Nach einer Nachrührzeit von 10 min. bei 35° C setzt man zunächst 10 g 50%ige Schwefelsäure, danach 106 g (1,25 Mol) Piperidin zu und preßt dann bei 40° C 60 bar Wasserstoff auf. Nach 4 Stunden erhöht man die Temperatur auf 90° C und den Wasserstoffdruck auf 70 bar, nach zwei weiteren Stunden die Temperatur auf 110° C und den $H_2$-Druck auf 90 bar (insgesamt ca. 8 Stunden). Man zieht unter Stickstoff ab und destilliert. Man erhält 293 g (1,07 Mol) einer Fraktion, die bei 0,2 mbar und 115 bis 117° C übergeht und reines 3[4-t-Butylphenyl]2-methylpropyl-piperidin darstellt [Umsatz, bezogen auf den Benzaldehyd, 95%; Selektivität 86%]. Der Katalysator kann für weitere Umsetzungen eingesetzt werden, ohne an Aktivität zu verlieren.

## Beispiel 4

In einem 4 l-Rührautoklaven werden 700 ml Ethanol, 50 g eines Katalysators, der 0,5% Palladium auf $\gamma$-$Al_2O_3$ enthält und 556 g (3,13 Mol) 4-t-Butoxybenzaldehyd unter Stickstoff vorgelegt, 17,5 g (0,44 Mol) NaOH zugefügt und 181 g (3,13 Mol) Propionaldehyd innerhalb von 6 Stunden bei 25 bis 30° C zugetropft.

Nach 15 Minuten Nachrühren wird mit 13,2 g Eisessig (0,22 Mol) versetzt und 4 Stunden bei 40° C und 30 bar Druck hydriert, danach noch bei 60° C ca. 2 Stunden. Man zieht ab und destilliert. Man erhält 577 g (2,62 Mol), 84% d. Th.) gaschromatographisch reines 3[4-Butoxyphenyl]-2-methyl-propanal. Der zurückbleibende Katalysator kann ohne weiteres wiederverwendet werden.

## Beispiel 5

Man legt in einem 2 l-Autoklaven 300 ml Methanol, 25 g eines 1%igen Palladium/Kohle-Katalysators und 400,5 g (2,25 Mol) 4-t-Butoxybenzaldehyd vor, fügt 56 g 30%ige methanolische Natriumethylatlösung zu, spült mit Stickstoff und fügt innerhalb von 5 Stunden 130 g (2,25 Mol) Propionaldehyd bei 25 bis 35° C zu. Nach 15 min werden 4,2 g (0,07 Mol) Eisessig zugesetzt und dann während 30 min 223 g (2,25 Mol) Perhydroazepin (Hexamethylenimin) bei 45° C zugegeben; es wird bei der gleichen Temperatur 50 bar Wasserstoff aufgepreßt, nach 4 Stunden auf 100° C erhitzt und bei 60 bar Druck die Umsetzung beendet. Nach dem Abziehen durch das Steigrohr mit Fritte erhält man 478 g (70% d. Th.) 3[4-t-Butoxyethoxy)phenyl]2-methylpropylperhydroazepin ($Kp_{0,1}$ = 118° C).

## Beispiel 6

In einem 1 l-Autoklaven werden 15 g Katalysator (0,5% Palladium auf $\gamma$-$Al_2O_3$), 400 ml Methanol, 6 g Natronlauge und 148 g (1 Mol) 4-Isopropylbenzaldehyd gefüllt. Man verschließt den Autoklaven, spült mit Stickstoff und setzt 58 g Propionaldehyd innerhalb von 4 Stunden bei 25° C zu.

Nach Neutralisation mit 50%iger Phosphorsäure wird dreimal mit Wasserstoff gespült, auf 20 bar Druck gebracht und bei 100° C in ca. 6 Stunden hydriert. Man zieht ab, destilliert und gewinnt 161,5 g einer Fraktion, die bei 135° C und 12 mbar siedet und analytisch und spektroskopisch reines 3[4-Isopro-

pylphenyl]2-methylpropanal darstellt.

Beispiel 7

In einem mit Stickstoff gespülten 1,2 l-Autoklaven, der ein Steigrohr mit Fritte besitzt, werden 9 g Katalysator (1% Palladium auf Rutil), 100 g Methanol, 10 g 50%ige KOH und 203 g 4-t-Butylbenzaldehyd (1,25 Mol) vorgelegt und innerhalb von 5 Stunden 61 g Acetaldehyd bei 20° C zugesetzt. Danach wird mit 5 ml Eisessig neutralisiert, dreimal mit $H_2$ gespült, 15 bar Wasserstoff augepreßt und bei 60° C und 20 bar bis zur Druckkonstanz hydriert. Über das Steigrohr wird die Reaktionslösung aus dem Autoklaven herausgepreßt; nach Abdestillieren des Lösungsmittels erhält man 166 g 3(4-t-Butylphenyl)propanal, das bei 0,3 mbar und 120 bis 130° C übergeht (70% Ausbeute).

**Patentansprüche**

1. Verfahren zur Herstellung von araliphatischen Aldehyden und/oder araliphatischen Aminen der Formel (I)

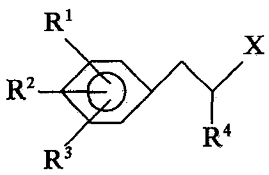

(I)

in der X den Rest $-CHO$ oder $-CH_2NR^5R^6$ bedeutet, wobei $R^1$, $R^2$ und $R^3$ gleich oder verschieden sein und je ein Wasserstoffatom, einen aliphatischen Rest mit bis zu 8 Kohlenstoffatomen, einen cycloaliphatischen Rest mit 4 bis 7 Kohlenstoffatomen, eine Alkoxygruppe mit bis zu 5 Kohlenstoffatomen, eine Hydroxylgruppe, eine tertiäre Aminogruppe, ein Halogenatom, einen gegebenenfalls mit bis zu 5 Kohlenstoffatomen substituierten Arylrest oder zu je zweien ein mit dem Benzolring kondensiertes weiteres Ringsystem bedeuten können, ferner $R^4$ ein Wasserstoffatom oder einen aliphatischen Rest mit bis zu 10 Kohlenstoffatomen sowie $R^5$ und $R^6$ je einen Alkylrest mit bis zu 10 Kohlenstoffatomen oder zusammen mit dem Stickstoffatom einen fünf-, sechs- oder siebengliedrigen heterocyclischen Ring bedeuten, der gegebenfalls ein weiteres Heteroatom wie Sauerstoff als Kettenglied enthält und gegebenenfalls seinerseits ein- oder mehrfach durch Alkylreste mit bis zu 4 Kohlenstoffatomen substituiert ist, durch Aldolkondensation eines gegebenenfalls substituierten Benzaldehyds der Formel (II) mit einem Alkanal der Formel (III)

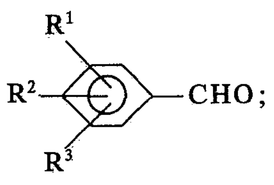

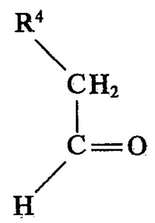

(II) (III)

in Gegenwart einer Base gegebenenfalls mindestens teilweiser Neutralisation und Umsetzung mit Wasserstoff (Hydrierung) und gegebenenfalls einem sekundären Amin der Formel $NHR^5R^6$, dadurch gekennzeichnet, daß die Umsetzungsprodukte der Aldolkondensation gegebenenfalls nach mindestens teilweiser Neutralisation unmittelbar der Hydrierung bzw. aminierenden Hydrierung mittels eines Palladiumkatalysators unterworfen werden.

2. Verfahren nach Anspruch 1, dadurch gekennzeichnet, daß die Aldolkondensation bei absatzweisem Betrieb in Gegenwart des Hydrierkatalysators vorgenommen wird.

3. Verfahren nach Anspruch 1, dadurch gekennzeichnet, daß als Hydrierkatalysator Palladium auf einem Aktivkohle, $SiO_2$, $Al_2O_3$ oder $TiO_2$ enthaltenden Träger verwendet wird.

4. Verfahren nach Anspruch 1, dadurch gekennzeichnet, daß man als Hydrierkatalysator Palladium im Gemisch mit Zink, Cadmium und/oder einem seltenen Erdmetall verwendet.

5. Verfahren nach einem der Ansprüche 1 bis 4, dadurch gekennzeichnet, daß die Hydrierung bzw. aminierende Hydrierung nach mindestens teilweiser Neutralisation der Base mit einer Carbonsäure, Sulfonsäure oder verdünnten halogenfreien Mineralsäure vorgenommen wird.

6. Verfahren nach einem der Ansprüche 1 bis 4, dadurch gekennzeichnet, daß die Aldolkondensation im wesentlichen bei bis zu 50° C, die Hydrierung bis zu 220° C und einem Wasserstoffdruck von bis zu 200 bar vorgenommen wird.

7. Verfahren nach einem der Ansprüche 1 bis 4, dadurch gekennzeichnet, daß man ein alkoholisches oder wäßrig-alkoholisches Lösungsmittel oder Wasser verwendet.

**Claims**

1. A process for the preparation of araliphatic aldehydes and/or araliphatic amines of the formula (I)

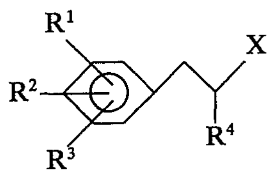

(I)

where X is —CHO or —$CH_2NR^5R^6$, $R^1$, $R^2$ and $R^3$ are identical or different and each can be hydrogen, an aliphatic radical of up to 8 carbon atoms, a cycloaliphatic radical of 4 to 7 carbon atoms, alkoxy of up to 5 carbon atoms, hydroxyl, tertiary amino, halogen or aryl which is unsubstituted or substituted by up to 5 carbon atoms, or any two radicals together can be a further ring system fused with the benzene ring, $R^4$ is hydrogen or an aliphatic radical of up to 10 carbon atoms, and $R^5$ and $R^6$ are each alkyl of up to 10 carbon atoms or, together with the nitrogen atom, are a five-membered, six-membered or seven-membered heterocyclic ring which may contain a further hetero-atom, eg. oxygen, as a chain member, and in turn is unsubstituted or monosubstituted or polysubstituted by alkyl of up to 4 carbon atoms, by aldol condensation of an unsubstituted or substituted benzaldehyde of the formula (II) with an alkanal of the formula (III)

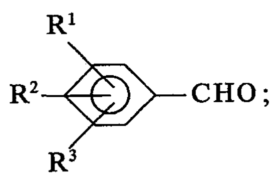

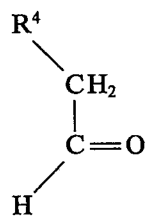

(II) (III)

in the presence of a base, with or without partial or complete neutralization, and reaction with hydrogen (hydrogenation), in the presence or absence of a secondary amine of the formula $NHR^5R^6$, wherein the reaction products of the aldol condensation, with or without partial or complete neutralization, are immediately hydrogenated, optionally under aminating conditions, in the presence of a palladium catalyst.

2. A process as claimed in claim 1, wherein, in batch operation, the aldol condensation is carried out in the presence of the hydrogenation catalyst.

3. A process as claimed in claim 1, wherein palladium on a carrier containing active charcoal, $SiO_2$, $Al_2O_3$ or $TiO_2$ is used as the hydrogenation catalyst.

4. A process as claimed in claim 1, wherein palladium mixed with zinc, cadmium and/or a rare earth metal is used as the hydrogenation catalyst.

5. A process as claimed in any of claims 1 to 4, wherein the hydrogenation, optionally under aminating conditions, is carried out after partial or complete neutralization of the base with a carboxylic acid, sulfonic acid or dilute halogen-free mineral acid.

6. A process as claimed in any of claims 1 to 4, wherein the aldol condensation is essentially carried out at up to 50° C, and the hydrogenation is carried out at up to 220° C and under a hydrogen pressure of up to 200 bar.

7. A process as claimed in any of claims 1 to 4, wherein an alcoholic or aqueous solvent or water is used.

**Revendications**

1. Procédé de préparation d'aldéhydes araliphatiques et/ou d'amines araliphatiques de formule I

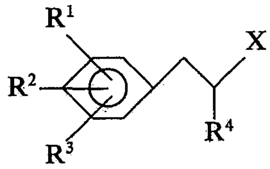

(I)

dans laquelle X représente le groupe $-CHO$ ou $-CH_2NR^5R^6$ dans lequel $R^1$, $R^2$ et $R^3$, ayant des significations identiques ou différentes, représentent chacun un atome d'hydrogène, un reste aliphatique contenant jusqu'à 8 atomes de carbone, un reste cycloaliphatique contenant 4 à 7 atomes de carbone, un groupe alcoxy contenant jusqu'à 5 atomes de carbone, un groupe hydroxy, un groupe amino tertiaire, un atome d'halogène, un groupe aryle portant éventuellement des substituants qui contiennent jusqu'à 5 atomes de carbone, ou bien deux de ces symboles représentent un autre système cyclique condensé avec le cycle benzénique, $R^4$ représente un atome d'hydrogène ou un reste aliphatique contenant jusqu'à 10 atomes de carbone, et $R^5$ et $R^6$ représentent chacun un groupe alkyle contenant jusqu'à 10 atomes de carbone ou forment ensemble et avec l'atome d'azote un noyau hétérocyclique à 5, 6 ou 7 chaînons, contenant éventuellement un autre hétéroatome comme l'oxygène en chaînon et qui peut lui-même être substitué le cas échéant une ou plusieurs fois par des groupe alkyle contenant jusqu'à 4 atomes de carbone, par condensation aldolique d'un benzaldéhyde éventuellement substitué de formule II avec un alcanal de formule III

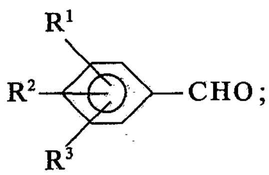

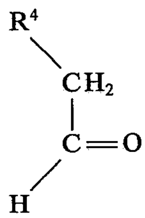

(II) (III)

en présence d'une base, neutralisation éventuelle, au moins partielle, et réaction avec l'hydrogène (hydrogénation) et éventuellement avec une amine secondaire de formule $NHR^5R^6$, caractérisé en ce que les produits de réaction de la condensation aldolique, éventuellement après neutralisation au moins partielle, sont soumis directement à l'hydrogénation ou l'hydrogénation aminante sur un catalyseur au palladium.

2. Procédé selon la revendication 1, caractérisé en ce que, en opération discontinue, la condensation aldolique est effectuée en présence du catalyseur d'hydrogénation.

3. Procédé selon la revendication 1, caractérisé en ce que l'on utilise en tant que catalyseur d'hydrogénation le palladium sur un support contenant du charbon actif, $SiO_2$, $Al_2O_3$ ou $TiO_2$.

4. Procédé selon la revendication 1, caractérisé en ce que l'on utilise en tant que catalyseur d'hydrogénation le palladium en mélange avec du zinc, du cadmium et/ou un métal des terres rares.

5. Procédé selon l'une quelconque des revendications 1 à 4, caractérisé en ce que l'on effectue l'hydrogénation ou l'hydrogénation aminante après neutralisation au moins partielle de la base par un acide carboxylique, un acide sulfonique ou un acide minéral non halogéné et dilué.

6. Procédé selon l'une quelconque des revendications 1 à 4, caractérisé en ce que l'on effectue la condensation aldolique essentiellement à des températures allant jusqu'à 50° C, l'hydrogénation à des températures allant jusqu'à 220° C et à une pression d'hydrogène allant jusqu'à 200 bars.

7. Procédé selon l'une quelconque des revendications 1 à 4, caractérisé en ce que l'on utilise un solvant alcoolique ou hydro-alcoolique ou l'eau.